# FASCICULE DE BREVET EUROPEEN

(11) **EP 1 587 492 B1**
(45) Date de publication et mention de la délivrance du brevet: **11.03.2009**
(21) Numéro de dépôt: 03717354.9
(22) Date de dépôt: 24.01.2003
(51) Int. Cl.: A61K 8/19, A61K 8/96, A61Q 19/00

(54) **COMPOSITIONS COSM ETIQUES NOTAMMENT EXFOLIANTES OU DE GOMMAGE CUTANE, COMPORTANT DE LA POUZZOLANE, ET LEURS APPLICATIONS**
KOSMETISCHE ZUSAMMENSETZUNGEN ZUM ENTFERNEN VON HAUTSCHUPPEN ENTHALTEND PUZZOLANE UND IHRE ANWENDUNGEN
COSMETIC COMPOSITIONS, PARTICULARLY EXFOLIANTS OR CUTANEOUS PACKS, COMPRISING POZZOLANA AND APPLICATIONS THEREOF

(43) Date de publication de la demande: 26.10.2005
(73) Titulaire: Gatellier, Nathalie, 63000 Clermont-Ferrand (FR)
(72) Inventeur: Gatellier, Nathalie, 63000 Clermont-Ferrand (FR)
(74) Mandataire: Myon, Gérard Jean-Pierre
(86) Numéro de dépôt international: PCT/FR2003/000242
(87) Numéro de publication internationale: WO 2004/073676

(56) Documents cités:
- EP-A- 0 336 900
- EP-A- 0 490 103
- CH-A- 692 409
- DE-B- 1 288 749

## Description

### Secteur technique de l'invention :

La présente invention concerne le secteur technique des compositions cosmétiques et de parapharmacie, notamment celles dénommées exfoliantes ou de gommage cutané.

### Art antérieur :

On rappellera que le gommage cutané ou exfoliant est l'action de destruction de la couche cornée de l'épiderme avec abrasion des cellules mortes, provoquant un phénomène de lissage de la couche superficielle de l'épiderme, à la différence du peeling qui provoque une réaction inflammatoire dermique.

Les ingrédients cosmétiques existant actuellement en tant qu'agents gommants sont regroupés en 2 catégories :
1- Gommants de synthèse (dérivés de polyéthylène).
2- Gommants naturels

### - Dérivés de produits végétaux

Généralement des parties végétales dures comme certaines coques de fruits (abricot, noisette) mais aussi certain exudats de plantes comme le bambou.

### - Dérivés de produits minéraux

La silice ou terre de diatomée.

Les principaux inconvénients de l'utilisation de ces agents gommants sont généralement liés à leur structure.

Par exemple, la poudre de noyaux d'abricot ou d'amande possède une dureté très faible par comparaison avec celle des produits minéraux gommants.
D'autre part, les risques microbiologiques sur la matière organique sont plus importants que sur les matières minérales.
De ce fait beaucoup d'ingrédients cosmétiques végétaux utilisés comme gommants subissent des traitements de stérilisation traumatisants (ionisation ou chaleur) qui réduisent l'activité de ces matières premières. DE-1288 749, CH- 692 409, EP-336 900 et EP- 490 104 divulguent des compositions cosmétiques exfoliantes contenant de la pierre de pouce

Les ingrédients minéraux possèdent une dureté plus élevée par rapport aux ingrédients végétaux, les risques micro biologiques sont aussi moins importants, la stérilisation compte tenu de leur composition chimique stable, est moins traumatisante, mais les produits connus possèdent néanmoins ces inconvénients, quoique à un degré moindre que les ingrédients végétaux.

### Problème technique posé :

La résultante de tous ces inconvénients est de rechercher un composé naturel minéral à fort pouvoir gommant ou exfoliant. Il doit agir de façon mécanique par sa dureté.

La Demanderesse s'est non seulement posé le problème ci-dessus, mais a aussi entrepris de se poser un problème entièrement original en lui-même, qui est de rechercher une capacité à apporter dans le milieu où il trouve (base cosmétique), puis vers la peau, une partie de son contenu minéral, oligo-éléments, et autres principes bénéfiques ou actifs, sous une forme apte à franchir la barrière cutanée.

Le produit recherché doit naturellement être apte à s'intégrer à des préparations cosmétiques ou de para-pharmacie, voire à des « médicaments » dans le cas (voir ci-dessous) où l'on adjoindrait de tels médicaments.

Compte tenu de l'absence d'avancées dans les produits gommants apportant plus que leur action exfoliante, il existe un besoin emportant, identifié pour la première fois par la Demanderesse, pour une composition à fort pouvoir gommant ou exfoliant qui, outre son action mécanique, serait de nature à apporter un avantage supplémentaire primordial sur les produits actuels, c'est-à-dire apporter au moins une partie de son contenu minéral.

### Résumé de l'invention :

L'invention repose sur l'incorporation de pouzzolane dans des compositions cosmétiques ou de para-pharmacie à action exfoliante ou de gommage cutané, en tant qu'élément actifs.

Il a été découvert de manière totalement surprenante que cet ingrédient, employé dans des conditions précises qui seront définies ci-après, non seulement procure une forte action de gommage cutané ou exfoliante, sans irriter la peau (pas de phénomène de rougeurs que l'on observe avec certains produits de gommage cutané ou de « peeling ») mais aussi apporte à la peau au moins une partie de son contenu minéral sous une forme bénéfique, notamment lorsqu'il est employé en combinaison avec des eaux thermales également chargées en minéraux bénéfiques.
La pouzzolane est une roche volcanique d'origine basaltique de composition chimique définie. Elle est de couleur ocre, rouge, grise et noire. Son utilisation connue est essentiellement en tant que :
Composé de sablage des routes
Matériaux de remblais
Confection de matériaux réfractaires
Confection de matériaux isolants phoniques et thermiques
Matériaux de drainage dans l'agriculture et dans l'horticulture.

### Description détaillée de l'invention :

Les compositions cosmétiques, de para-pharmacie et (lorsqu'elles contiennent de plus au moins une dose efficace d'un principe pharmaceutiquement actif) pharmaceutiques selon l'invention sont caractérisées en ce qu'elles contiennent comme principe actif de gommage cutané ou agent exfoliant au moins de la pouzzolane .

L'emploi de pouzzolane dans ce contexte a requis de nombreuses innovations techniques et recherches. On ne peut utiliser la pouzzolane sous son état naturel, d'une part, et d'autre part la granulométrie ne sera pas identique pour différentes formes de compositions comme une crème, un gel douche lavant et analogues.

Par ailleurs, il convenait d'obtenir le meilleur apport possible en minéraux.

Il a été établi que, pour obtenir les meilleures propriétés, il convient d'employer la pouzzolane à une granulométrie de 160 à 540 microns, de préférence 200 à 400 microns, avec éventuellement des plages encore préférées selon la forme de la composition (gel, crème, savon, etc...).

La pouzzolane a de multiples applications dans le domaine de la cosmétique en tant que gommant dans les produits de soin corporel, les produits de soins visage, les produits capillaires. Le pourcentage d'utilisation est variable suivant le type de produit, les plages variant de 0,1 % à 70 %en poids.

Selon la forme des compositions finales (gel, savon, etc...), on incorpore la pouzzolane à raison de 0,5 à 35 % en poids, de préférence 0,5 à 30%, de préférence 0,5 à 10 %, de préférence de 0,5 à 5 % et encore de préférence de 0,5 à 1 %.

Selon un mode de réalisation particulier, on incorpore également de l'eau thermale, notamment de la Région Auvergne.

Il a été découvert de manière surprenante que, non seulement la pouzzolane est capable de réaliser un apport d'au moins une partie de ses minéraux bénéfiques, mais que de plus son caractère absorbant permettait (sans vouloir être lié par une théorie spécifique) d'absorber les minéraux d'eaux thermales, puis de réaliser le même apport avec ces minéraux d'eau thermales qu'avec ses propres minéraux. La pouzzolane sert alors, dans ce cas, d'agent de transfert des minéraux des eaux thermales vers la peau en facilitant leur passage de la barrière cutanée.

La Demanderesse évoque cette possibilité, après avoir constaté les propriétés obtenues, en raison, a posteriori, de la structure connue, scoriacée alvéolaire, de la pouzzolane. Cependant, d'autres produits a priori poreux ne donnent pas les mêmes résultats.

On pense qu'il se produit une réaction d'échange, probablement à caractère ionique ou par réalisation de mailles, d'effet chélatant et phénomènes analogues, entre la pouzzolane et ladite base cosmétique ; les minéraux de la pouzzolane qu'elle contient naturellement, avec éventuellement les minéraux d'eau thermale contenue dans la base cosmétique, passent donc dans la base et de la base vers la peau. On pense également que ces minéraux et autres principes actifs ou bénéfiques (cf. ci-dessous) peuvent être modifiés par leur absorption - désorption par la pouzzolane, par exemple sous formes de chélates ou analogues, ce qui facilite leur passage de la barrière cutanée.

La capacité des ingrédients gommants connus à retenir un composé liquide est très faible, et leur participation aux échanges minéraux avec la peau est pratiquement nulle. Aucune recherche n'a donc été entreprise dans le passé dans cette direction, à notre connaissance, ou du moins aucun résultat n'a été obtenu.

Ayant découvert ce principe original, l'invention couvre également l'incorporation de tous composants compatibles avec les applications cosmétiques et de para-pharmacie, et contenant de tels minéraux ou principes actifs ou principes bénéfiques capables d'être absorbés par la pouzzolane.

Selon un mode de réalisation particulier, l'invention incorpore éventuellement des extraits de plantes médicinales, des extraits de plantes aromatiques, des huiles essentielles de telles plantes, éventuellement de l'eau thermale et /ou de l'eau de source(s) minérale(s) et /ou à des eaux ou solutions salines spécialement chargées en certains minéraux, par exemple eau thermale additionnée de magnésium, et/ou de soufre, ou autres principes actifs reconnus pour leur aspect bénéfique (le soufre étant par exemple utile contre les maladies de peau comme l'acné juvénile ou analogue). Certaines de ces formulations, de par l'addition de « médicaments » seront elles-mêmes des médicaments réservés à la prescription par un médecin.

Les extraits de plantes et huiles essentielles sont de préférence choisis parmi :
la camomille, lavande, lavandin, cyprès, pin, sapin, citron, algues, extraits de plantes médicinales, extraits de plantes aromatiques, extraits de fruits et légumes, extraits d'algues, sel marin, argile, minéraux et vitamines.

L'homme du métier saura naturellement employer tous les extraits de plantes et huiles essentielles reconnus comme bénéfiques et apportant des sels et oligo-éléments.

Les oligo-éléments sont ceux classiques: fer, magnésium, manganèse, zinc, cuivre, etc.... qu'il est inutile de détailler. On peut ajouter le soufre, utile contre les maladies bénignes de la peau.

La ou les eaux thermales employées sont utilisées à raison de 0 à la quantité QSP c'est-à-dire permettant de compléter la composition cosmétique à 100 % en poids.

Il n'existe pas de limite supérieure particulière.

On pourra employer toute eau thermale ou de source, selon l'effet souhaité.

Il sera préférable d'employer au moins 5 % en poids, ou de préférence au moins 10 - 20 %, ou de préférence 50 à 70 - 100 % en poids d'eau(x) thermale(s) et / ou de source(s) et / ou solutions salines dans la partie aqueuse de la composition.

De même, les extraits de plantes aromatiques, extraits de plantes médicinales, huiles essentielles, seront de préférence présentes à raison (chacune) de 0 à 1 ou 2 % en poids, de préférence de 0 à 3 ou 4 % en poids de la composition cosmétique.

Dans tous les cas, on ajuste la quantité d'eau (soit déminéralisée, soit déminéralisée et/ou thermale(s) et / ou de source(s) et / ou solution saline) à la quantité QSP 100 %.

L'homme de métier saura composer des compositions variées, présentant des senteurs et propriétés variées d'apport minéral et d'apport de principes actifs et de principes bénéfiques, ainsi qu'un effet de gommage plus ou moins important, en faisant varier selon « les bonnes pratiques » cosmétiques et pharmaceutiques les proportions des ingrédients, ainsi que la granulométrie de la pouzzolane. De même, ces proportions et granulométrie seront adaptées par l'homme de métier à la composition finale, c'est-à-dire par exemple crème, savon, gel douche, etc...., une granulométrie plus fine étant par exemple préférée pour une crème, et une granulométrie plus grossière pour un savon.

L'homme de métier comprendra que l'on incorpore également, le cas échéant, des parfums et autres additifs classiques en cosmétiques.

Cependant, le choix des plantes et huiles essentielles confère déjà une senteur naturelle qu'il sera éventuellement très souhaitable de conserver intacte.

On agrémentera par exemple les compositions selon trois axes principaux : axe Forêt, axe Fleurs, axe Fruits, afin de relier les produits aux caractères de la terre (au sens large, c'est-à-dire y compris les plantes qui y poussent) dont les principes actifs sont extraits.

On peut aussi avoir recours à différents arômes.

L'invention couvre toutes les formes exfoliantes ou de gommage cutané pharmaceutiquement acceptables, et acceptables en cosmétologie, aussi bien pour l'usage par les particuliers que par les médecins en cosmétologie, par exemple :
- gel douche exfoliant, gel exfoliant pour le visage, pour le corps, huile exfoliante, lait fluide exfoliant, crème mousse exfoliante (ces formes d'application sont originales en elles mêmes)
- savons exfoliants, savons-crème exfoliants,
- avec les adaptations dont est facilement capable l'homme de métier selon par exemple qu'il s'agir d'une application pour un particulier, c'est-à-dire « grand-public » ou d'une application pour un médecin, la formulation pouvant par exemple dans ce dernier cas présenter un pouvoir gommant plus puissant, et ou être additionnée de principes actifs supplémentaires dont l'usage est réservé aux médecins, voire de certains médicaments compatibles avec le reste de la formulation.

L'invention couvre également lesdits médicaments, caractérisés en ce qu'ils se présentent sous la forme d'une composition selon l'invention, comportant de plus au moins une dose efficace d'un principe pharmaceutiquement actif compatible avec ladite composition et son mode d'application.

La préparation des formulations selon l'invention se fait par mélange classique des ingrédients, selon les procédés connus de compositions exfoliantes, avec deux options :
- soit la pouzzolane et les divers principes actifs éventuels (eaux thermales, eau de source, huiles essentielles, extraits de plantes, solutions salines, etc...) sont mélangés ensemble,
- soit la pouzzolane est imprégnée auparavant de tout ou partie de ces éventuels principes actifs, afin de maximiser l'absorption.

L'invention sera mieux comprise à la lecture de la description qui va suivre, et des exemples non limitatifs ci-dessous.

### EXEMPLES :

| GEL EXFOLIANT | | % |
|---|---|---|
| Eau déminéralisée ou eau de source(s) | | et/ou eau(x) thermale(s) |
| | QSP | 100 |
| Actif végétaux | | 20 |
| Carbomer | | 0,4 |
| Xanthan gum | | 0,8 |
| Pouzzolane (200-400 µm) | | 2 |
| Huile essentielle | | 0,45 |
| Conservateurs | | 0,5 |
| Hydroxyde sodium | | 0,2 |

Les plages d'utilisation sont comprises dans cette formulation de 0,5 à 5%

| GEL DOUCHE EXFOLIANT | | % |
|---|---|---|
| Eau déminéralisée ou eau de source(s) et/ou | | eau(x) thermale(s) |
| | QSP | 100 |
| Base lavante | | 39 |
| Actif végétaux | | 16 |
| Pouzzolane (260-400 µm) | | 5 |
| Conservateurs | | 0,5 |
| Huile essentielle | | 0,5 |
| Chlorure de sodium | | 2,2 |
| Acide citrique 10% | | 0,25 |

Les plages d'utilisation sont comprises dans cette formulation de 0,5 à 10%

| GEL DOUCHE EXFOLIANT enrichi en soufre 0,05% | | | |
|---|---|---|---|
| Eau(x) thermale(s) enrichies à 0,05 % en poids de soufre par addition de 0,5g/l de soufre sublimé | | | |
| | QSP | 100 | |
| Base lavante | | 39 | |
| Actif végétaux | | 16 | |
| Soufre sublimé | | 0,5 | |
| Pouzzolane (240-380 µm) | | 5 | |
| Conservateurs | | 0,5 | |
| Huile essentielle | | 0,5 | |
| Chlorure de sodium | | 2,2 | |
| Acide citrique 10% | | 0,25 | |

Les plages d'utilisation sont comprises dans cette formulation de 0,5 à 10%

| CREME MOUSSE EXFOLIANTE | % | |
|---|---|---|
| Eau Thermale de la Bourboule | QSP | 100 |
| Base autoémulsifiable | 4 | |
| Cetearyl alcohol | 4 | |
| Actif végétaux | 5 | |
| Pouzzolane (220-400 µm) | 1 | |
| Conservateurs | 0,5 | |
| Huile essentielle | 0,4 | |
| PEG-7 glyceryle cocoate | 1 | |

Les plages d'utilisation sont comprises dans cette formulation de 0,5 à 5%

| PAIN DE SAVON EXFOLIANT | | % |
|---|---|---|
| Base végétale | QSP | 100 |
| Pouzzolane (200-400 µm) | | 15 |
| Huile essentielle | | 0,5 |

PAIN DE SAVON EXFOLIANT enrichi en vitamines et magnésium A 0,1% de Vitamine B5, 0,1% de Vitamine B1 et 0,1% de sulfate de magnésium

| Base végétale | QSP | 100 |
|---|---|---|
| Sulfate de magnésium | | 2 |
| Vitamine B5 | | 0,1 |
| Vitamine B1 | | 0,1 |
| Pouzzolane (200-400 µm) | | 15 |
| Huile essentielle | | 0,5 |

Les plages d'utilisation sont comprises dans cette formulation de 1 à 30%

| PAIN DE SAVON EXFOLIANT enrichi en extraits d'algues et sel marin 1% | | |
|---|---|---|
| Base végétale | QSP | 100 |
| Pouzzolane (200-400 µm) | | 15 |
| Huile essentielle | | 0,5 |
| Extrait d'algues marines | | 0,4 |
| Sel marin (Ile de Noirmoutier) | | 1 |
| Pouzzolane (200-400 µm) | | 15 |
| Huile essentielle | | 0,5 |

Les plages d'utilisation sont comprises dans cette formulation de 1 à 30%

| HUILE EXFOLIANTE | % | |
|---|---|---|
| Huile | QSP | 100 |
| Gélifiant huileux | | 20 |
| Silicone volatile | | 10 |
| Principes Actifs végétaux | | 10 |
| Pouzzolane (200-400 µm) | | 1,5 |
| Conservateurs | | 0,5 |
| Huile essentielle | | 0,45 |

Les plages d'utilisation sont comprises dans cette formulation de 0,5 à 10%

| GEL CREME DOUCHE EXFOLIANT | % | |
|---|---|---|
| Eau déminéralisée | QSP | 100 |
| Base lavante | | 39 |
| Base autoémulsifiable | | 32 |
| Pouzzolane (200-400 µm) | | 1,5 |
| Conservateurs | | 0,5 |
| Huile essentielle | | 0,3 |
| Chlorure de sodium | | 2,2 |
| Acide citrique 10% | | 0,25 |

Les plages d'utilisation sont comprises dans cette formulation de 0,5 à 5%

### GEL CREME DOUCHE EXFOLIANT enrichi en sels minéraux et magnésium 0,1%

Solution de citrate de magnésium, citrate de zinc, citrate de potassium à 0,1 % de chaque oligo-élément, rapporté au poids de la composition totale

| | QSP | 100 |
|---|---|---|
| Base lavante | | 39 |
| Base autoémulsifiable | | 32 |
| Pouzzolane (200-400 µm) | | 1,5 |
| Citrate de magnésium | | 0,1 |
| Citrate de zinc | | 0,1 |
| Citrate de potassium | | 0,1 |
| Conservateurs | | 0,5 |
| Huile essentielle | | 0,3 |
| Chlorure de sodium | | 2,2 |
| Acide citrique 10% | | 0,25 |

Les plages d'utilisation sont comprises dans cette formulation de 0,5 à 5%

| LAIT | |
|---|---|
| QSP 100 | |
| Base autoémulsifiable | 32 |
| Agent épaississant | 2,5 |
| Emollient | 10 |
| Huile de vaseline codex | 15 |
| Gelifiant hydrophyle | 0,3 |
| Soude | 0,2 |
| Pouzzolane (200-400 µm) | 2 |
| Conservateurs | 0,5 |
| Huile essentielle | 0,3 |

Les plages d'utilisation sont comprises dans cette formulation de 0,5 à 5%

L'invention couvre également tous les modes de réalisation et toutes les applications qui seront directement accessibles à l'homme de métier à la lecture de la présente demande, de ses connaissances propres, et éventuellement d'essais simples de routine.

## Revendications

1. Compositions cosmétiques ou de para-pharmacie ou pharmaceutiques à action exfoliante ou de gommage cutané, **caractérisées en ce que** elles comportent au moins de la pouzzolane en tant qu'élément actif gommant ou exfoliant.

2. Compositions selon la revendication 1 **caractérisées en ce qu'**elles contiennent la pouzzolane à une granulométrie de 160 à 540 microns, de préférence 200 à 400 microns.

3. Compositions selon la revendication 1 ou 2 **caractérisées en ce que** le pourcentage de pouzzolane ou est compris entre 0,1 % et 70 % en poids.

4. Compositions selon la revendication 3 **caractérisées en ce qu'**on incorpore la pouzzolane à raison de 0,5 à 35 % en poids, de préférence 0,5 à 30 %, de préférence 0,5 à 10 %, de préférence de 0,5 à 5 % et encore de préférence de 0,5 à 1 %.

5. Compositions selon l'une quelconque des revendications 1 à 4 **caractérisées en ce qu'**elles comprennent également des composants compatibles avec les applications cosmétiques et de para-pharmacie ou pharmacie, et contenant de tels minéraux ou principes actifs ou principes bénéfiques capables d'être absorbés par la pouzzolane.

6. Compositions selon la revendication 5 **caractérisées en ce qu'**on y incorpore des composants choisis, seuls ou en combinaison, parmi :
- des extraits de plantes médicinales,
- des extraits de plantes aromatiques,
- des huiles essentielles de telles plantes,
- de l'eau thermale et /ou de l'eau de source(s) minérale(s) .
- des eaux ou solutions salines spécialement chargées en certains minéraux, par exemple eau thermale additionnée de magnésium, et/ou de soufre, ou autres principes.

7. Compositions selon la revendication 5 ou 6 **caractérisées en ce que** les extraits de plantes et huiles essentielles sont choisis parmi :
l'huile essentielle de camomille, lavande, lavandin, cyprès, pin, sapin, citron, algues, extraits de plantes médicinales, extraits de plantes aromatiques, extraits de fruits et légumes, extraits d'algues.

8. Compositions selon la revendication 5 ou 6, **caractérisées en ce qu'**elles comprennent, seuls ou en combinaison, du sel marin, de l'argile, des minéraux et des vitamines.

9. Compositions selon l'une quelconque des revendications 5 à 8 **caractérisées en ce que** la ou les eaux thermales et / de source et / ou les solutions salines employées sont utilisées à raison de 0 à la quantité QSP c'est-à-dire permettant de compléter la composition cosmétique à 100 % en poids.

10. Compositions selon l'une quelconque des revendications 5 à 9 **caractérisées en ce qu'**elles contiennent au moins 5 % en poids, ou de préférence au moins 10 - 20 %, ou de préférence 50 à 70 - 100 % en poids d'eau(x) thermale(s) ou de source(s) dans la partie aqueuse de la composition.

11. compositions selon l'une quelconque des revendications 5 à 10 **caractérisées en ce que** les extraits de plantes aromatiques, et de plantes médicinales, huiles essentielles, sont présentes à raison (chacune) de 0 à 1 ou 2 % en poids, de préférence de 0 à 3 ou 4 % en poids de la composition cosmétique.

12. Compositions selon l'une quelconque des revendications 1 à 11 **caractérisées en ce qu'**on ajuste la quantité d'eau (soit déminéralisée, soit déminéralisée et/ou thermale(s) et / ou de source(s) et ou solution saline) à la quantité QSP 100 %.

13. Compositions selon l'une quelconque des revendications 1 à 12 **caractérisées en ce qu'**elles se présentent sous des formes exfoliantes ou de gommage cutané pharmaceutiquement acceptables, et acceptables en cosmétologie, aussi bien pour l'usage par les particuliers que par les médecins en cosmétologie, choisies parmi les formes suivantes :
- gel douche exfoliant, gel exfoliant pour le visage, pour le corps, huile exfoliante, lait fluide exfoliant, crème mousse exfoliante
- savons exfoliants, savons-crème exfoliants,
- ces mêmes formes d'application contenant des médicaments compatibles avec le reste de la formulation.

14. Médicaments **caractérisés en ce qu'**ils se présentent sous la forme d'une composition selon l'une, quelconque des revendications 5 à 13 comportant de plus au moins une dose efficace d'un principe pharmaceutique ment actif compatible avec ladite composition et son mode d'application.

15. Procédé de préparation des compositions selon l'une quelconque des revendications 1 à 14 **caractérisé en ce qu'**on réalise un mélange des ingrédients, avec deux options :
- soit la pouzzolane et les divers principes actifs éventuels, eaux thermales, eau de source, huiles essentielles, extraits de plantes, solutions salines, sont mélangés ensemble,
- soit la pouzzolane est imprégnée auparavant de tout ou partie de ces éventuels principes actifs, afin de maximiser l'absorption.

16. Composition selon l'une quelconque des revendications 1 à 13, **caractérisée en ce qu'**elle consiste en un
| GEL EXFOLIANT | % |
|---|---|
| Eau déminéralisée ou eau de source(s) et/ou eau(x) thermale(s) | |
| QSP | 100 |
| Actif végétaux | 20 |
| Carbomer | 0,4 |
| Xanthan gum | 0,8 |
| Pouzzolane (200-400 µm) | 2 |
| Huile essentielle | 0,45 |
| Conservateurs | 0,5 |
| Hydroxyde sodium | 0,2 |

17. Composition selon l'une quelconque des revendications 1 à 13, **caractérisée en ce qu'**elle consiste en un
| GEL DOUCHE EXFOLIANT | | % |
|---|---|---|
| Eau déminéralisée ou eau de source(s) et/ou eau(x) thermale(s) | | |
| | QSP | 100 |
| Base lavante | | 39 |
| Actif végétaux | | 16 |
| Pouzzolane (260-400 µm) | | 5 |
| Conservateurs | | 0,5 |
| Huile essentielle | | 0,5 |
| Chlorure de sodium | | 2,2 |
| Acide citrique 10% | | 0,25 |

18. Composition selon l'une quelconque des revendications 1 à 13, **caractérisée en ce qu'**elle consiste en une
| CREME MOUSSE EXFOLIANTE | % | |
|---|---|---|
| Eau Thermale de la Bourboule | QSP | 100 |
| Base autoémulsifiable | 4 | |
| Cetearyl alcohol | 4 | |
| Actif végétaux | 5 | |
| Pouzzolane (220-400 µm) | 1 | |
| Conservateurs | 0,5 | |
| Huile essentielle | 0,4 | |
| PEG-7 glyceryle cocoate | 1 | |

19. Composition selon l'une quelconque des revendications 1 à 13, **caractérisée en ce qu'**elle consiste en un
| PAIN DE SAVON EXFOLIANT | % | |
|---|---|---|
| Base végétale | QSP | 100 |
| Pouzzolane (200-400 µm) | | 15 |
| Huile essentielle | | 0,5 |
| PAIN DE SAVON EXFOLIANT enrichi en vitamines et magnésium | |
|---|---|
| A 0.1% de Vitamine B5, 0,1% de Vitamine B1 et 0,1% de sulfate de magnésium | |
| Base végétale | QSP 100 |
| Sulfate de magnésium | 2 |
| Vitamine B5 | 0,1 |
| Vitamine B1 | 0,1 |
| Pouzzolane (200-400 µm) | 15 |
| Huile essentielle | 0,5 |

20. Composition selon l'une quelconque des revendications 1 à 13, **caractérisée en ce qu'**elle consiste en un
| PAIN DE SAVON EXFOLIANT enrichi en extraits d'algues et sel marin 1% | | |
|---|---|---|
| Base végétale | QSP | 100 |
| Pouzzolane (200-400 µm) | | 15 |
| Huile essentielle | | 0,5 |
| Extrait d'algues marines | | 0,4 |
| Sel marin (Ile de Noirmoutier) | | 1 |
| Pouzzolane (200-400 µm) | | 15 |
| Huile essentielle | | 0,5 |

21. Composition selon l'une quelconque des revendications 1 à 13, **caractérisée en ce qu'**elle consiste en une
| HUILE EXFOLIANTE | % | |
|---|---|---|
| Huile | QSP | 100 |
| Gélifiant huileux | 20 | |
| Silicone volatile | 10 | |
| Principes Actifs végétaux | 10 | |
| Pouzzolane (200-400 µm) | 1,5 | |
| Conservateurs | 0,5 | |
| Huile essentielle | 0,45 | |

22. Composition selon l'une quelconque des revendications 1 à 13, **caractérisée en ce qu'**elle consiste en un
| GEL CREME DOUCHE EXFOLIANT | % | |
|---|---|---|
| Eau déminéralisée | QSP | 100 |
| Base lavante | 39 | |
| Base autoémulsifiable | 32 | |
| Pouzzolane (200-400 µm) | 1,5 | |
| Conservateurs | 0,5 | |
| Huile essentielle | 0,3 | |
| Chlorure de sodium | 2,2 | |
| Acide citrique 10% | 0,25 | |

23. Composition selon l'une quelconque des revendications 1 à 13, **caractérisée en ce qu'**elle consiste en un
| GEL CREME DOUCHE EXFOLIANT enrichi en sels minéraux et magnésium 0,1% | | |
|---|---|---|
| Solution de carbonate de magnésium, sulfate de zinc, saccharinate de sodium, citrate de potassium à 0,1 % de chaque oligo-élément, rapporté au poids de la composition totale | | |
| | QSP | 100 |
| Base lavante | | 39 |
| Base autoémulsifiable | | 32 |
| Pouzzolane (200-400 µm) | | 1,5 |
| Citrate de magnésium | | 0,1 |
| Citrate de zinc | | 0,1 |
| Citrate de potassium | | 0,1 |
| Conservateurs | | 0,5 |
| Huile essentielle | | 0,3 |
| Chlorure de sodium | | 2,2 |
| Acide citrique 10% | | 0,25 |

24. Composition selon l'une quelconque des revendications 1 à 13, **caractérisée en ce qu'**elle consiste en un
| LAIT | |
|---|---|
| QSP 100 | |
| Base autoémulsifiable | 32 |
| Agent épaississant | 2,5 |
| Emollient | 10 |
| Huile de vaseline codex | 15 |
| Gelifiant hydrophyle | 0,3 |
| Soude | 0,2 |
| Pouzzolane (200-400 µm) | 2 |
| Conservateurs | 0,5 |
| Huile essentielle | 0,3 |

## Claims

1. Cosmetic or para-pharmaceutical or exfoliating action pharmaceutical or skin scrub compositions, **characterised in that** they comprise at least pozzolana as an active scrub or exfoliating element.

2. Compositions according to claim 1, **characterised in that** they contain pozzolana with a particle size distribution of 160 to 540 microns, preferably 200 to 400 microns.

3. Compositions according to claim 1 or 2, **characterised in that** the percentage of pozzolana is comprised between 0.1 wt.% and 70 wt.%.

4. Compositions according to claim 3, **characterised in that** the pozzolana is incorporated at a rate of 0.5 to 35 wt.%, preferably 0.5 to 30%, preferably 0.5 to 10%, preferably 0.5 to 5% and more preferably 0.5 to 1%.

5. Compositions according to any one of claims 1 to 4, **characterised in that** they also comprise components compatible with the cosmetic and para-pharmaceutical or pharmaceutical applications, and containing minerals or active ingredients or beneficial ingredients capable of being absorbed by the pozzolana.

6. Compositions according to claim 5, **characterised in that** selected components are incorporated therein, singly or in combination, including:
extracts of medicinal plants,
- extracts of aromatic plants,
- essential oils of such plants,
- thermal water and/or water from mineral source(s)
- saline solutions or waters specially charged with certain minerals, for example thermal water to which magnesium and/or sulfur or other ingredients have been added.

7. Compositions according to claim 5 or 6, **characterised**
**in that** the plant extracts and essential oils are selected from:
the essential oil of camomile, lavender, lavandin, cypress, pine, fir, lemon, seaweed, extracts of medicinal plants, extracts of aromatic plants, extracts of fruits and vegetables and extracts of seaweed.

8. Compositions according to claim 5 or 6, **characterised in that** they comprise, singly or in combination, sea salt, clay, minerals and vitamins.

9. Compositions according to any one of claims 5 to 8, **characterised in that** the thermal and/or spring water or waters and/or saline solutions employed are used at a rate of 0 to QS, that is, allowing the cosmetic composition to be made up to 100 wt.%.

10. Compositions according to any one of claims 5 to 9, **characterised in that** they contain at least 5 wt.%, or preferably at least 10 - 20 wt.%, or preferably 50 to 70 - 100 wt.% of thermal or spring water(s) in the aqueous part of the composition.

11. Compositions according to any one of claims 5 to 10, **characterised in that** the extracts of aromatic plants and of medicinal plants, essential oils, are present at a rate (each) of 0 to 1 or 2 wt.%, preferably 0 to 3 or 4 wt.% of the cosmetic composition.

12. Compositions according to any one of claims 1 to 11, **characterised in that** the quantity of water (i.e.deionised, deionised and/or thermal and and/or spring and/or saline solution) at QS 100% is adjusted.

13. Compositions according to any one of claims 1 to 12, **characterised in that** they occur in pharmaceutically acceptable, and acceptable in cosmetology, exfoliating or skin scrub forms, both for use by individuals and by cosmetic doctors, selected from the following forms;
- exfoliating shower gel, exfoliating gel for the face, for the body, exfoliating oil, exfoliating fluid milk, exfoliating cream foam
- exfoliating soaps, exfoliating cream soaps,
- these same application forms, containing medicinal products compatible with the rest of the formulation.

14. Medicinal products, **characterised in that** they occur in the form of a composition according to any one of claims 5 to 13, comprising furthermore at least one effective dose of a pharmaceutically active ingredient compatible with the said composition and its mode of application.

15. Process for the preparation of the compositions according to any one of claims 1 to 14, **characterised**
**in that** a mixture of the ingredients is carried out, with two options:
- either pozzolana and the various possible active ingredients, thermal waters, spring water, essential oils, plant extracts, saline solutions are mixed together,
- or the pozzolana is impregnated previously with all or some of these possible active ingredients, in order to maximise absorption.

16. Composition according to any one of claims 1 to 13, **characterised in that** it consists of an
| EXFOLIATING GEL | % |
|---|---|
| Deionised water or spring water(s) | water(s) and/or thermal |
| QS | 100 |
| Plant active ingredient | 20 |
| Carbomer | 0.4 |
| Xanthan gum | 0.8 |
| Pozzolana (200 - 400 µm) | 2 |
| Essential oil | 0.45 |
| Preservatives | 0.6 |
| Sodium hydroxide | 0.2 |

17. Composition according to any one of claims 1 to 13, **characterised in that** it consists of an
| EXFOLIATING SHOWER GEL % |
|---|
| Deionised water or spring water(s) and/or thermal water(s) |
| QS 100 |
| Cleansing base 39 |
| Plant active ingredient 16 |
| Pozzolana (260 - 400 µm) 5 |
| Preservatives 0.5 |
| Essential oil 0.5 |
| Sodium chloride 2.2 |
| Citric acid 10% 0.25 |

18. Composition according to any one of claims 1 to 13, **characterised in that** it consists of an
| | | |
|---|---|---|
| EXFOLIATING CREAM FOAM | % | |
| Bourboule thermal water | QS | 100 |
| Self-emulsifiable base | 4 | |
| Cetearyl alcohol | 4 | |
| Plant active ingredient | 5 | |
| Pozzolana (220 - 400 µm) | 1 | |
| Preservatives | 0.5 | |
| Essential oil | 0.4 | |
| PEG-7 glyceryl cocoate | 1 | |

19. Composition according to any one of claims 1 to 13, **characterised in that** it consists of an
| EXFOLIATING SOAP BAR | %- | |
|---|---|---|
| Plant base | QS | 100 |
| Pozzolana (200 - 400 µm) | | 15 |
| Essential oil | | 0.5 |
EXFOLIATING SOAP BAR enriched with vitamins and magnesium
with 0.1% vitamin B5, 0.1% vitamin B1 and 0.1% magnesium sulfate
| Plant base | QS | 100 |
|---|---|---|
| Magnesium sulfate | | 2 |
| Vitamin B5 | | 0.1 |
| Vitamin B1 | | 0.1 |
| Pozzolana (200 - 400 µm) | | 15 |
| Essential oil | | 0.5 |

20. Composition according to any one of claims 1 to 13, **characterised in that** it consists of an
EXFOLIATING SOAP BAR enriched with extracts of seaweed and sea salt 1%
| Plant base | QS | 100 |
|---|---|---|
| Pozzolana (200 - 400 µm) | | 15 |
| Essential oil | | 0.5 |
| Seaweed extracts | | 0.4 |
| Sea salt (Ile de Noirmoutier) | | 1 |
| Pozzolana (200 - 400 µm) | | 15 |
| Essential oil | | 0.5 |

21. Composition according to any one of claims 1 to 13, **characterised in that** it consists of an
| EXFOLIATING OIL | % | |
|---|---|---|
| Oil | QS | 100 |
| Oily gelling agent | 20 | |
| Volatile silicone | 10 | |
| Plant active ingredients | 10 | |
| Pozzolana (200 - 400 µm) | 1.5 | |
| Preservatives | 0.5 | |
| Essential oil | 0.45 | |

22. Composition according to any one of claims 1 to 13, **characterised in that** it consists of an
| EXFOLIATING CREAM SHOWER GEL | % | |
|---|---|---|
| Deionised water | QS | 100 |
| Cleansing base | 39 | |
| Self-emulsifiable base | 32 | |
| Pozzolana (200 - 400 µm) | 1.5 | |
| Preservatives | 0.5 | |
| Essential oil | 0.3 | |
| Sodium chloride | 2.2 | |
| Citric acid 10% | 0.25 | |

23. Composition according to any one of claims 1 to 13, **characterised in that** it consists of an
EXFOLIATING CREAM SHOWER GEL enriched with mineral salts and magnesium 0.1%
Solution of magnesium carbonate, zinc sulfate, sodium saccharinate, 0.1% potassium citrate for each oligo element, related to the weight of the total composition
| | QS | 100 |
|---|---|---|
| Cleansing base | | 39 |
| Self-emulsifiable base | | 32 |
| Pozzolana (200 - 400 µm) | | 1.5 |
| Magnesium citrate | | 0.1 |
| Zinc citrate | | 0.1 |
| Potassium citrate | | 0.1 |
| Preservatives | | 0.5 |
| Essential oil | | 0.3 |
| Sodium chloride | | 2.2 |
| Citric acid 10% | | 0.25 |

24. Composition according to any one of claims 1 to 13, **characterised in that** it consists of a
| | |
|---|---|
| MILK | |
| QS 100 | |
| Self-emulsifiable base | 32 |
| Thickening agent | 2.5 |
| Emollient | 10 |
| Vaseline oil codex | 15 |
| Hydrophilic gelling agent | 0.3 |
| Soda | 0.2 |
| Pozzolana (200 - 400 µm) | 2 |
| Preservatives | 0.5 |
| Essential oil | 0.3 |

## Patentansprüche

1. Kosmetische, parapharmazeutische oder pharmazeutische Zusammensetzungen mit exfolierender oder abrasiver Wirkung gegenüber der Haut, **dadurch gekennzeichnet, dass** sie mindestens Puzzolanerde als abrasiven oder exfolierenden Wirkstoff umfassen.

2. Zusammensetzungen nach Anspruch 1, **dadurch gekennzeichnet, dass** sie Puzzolanerde mit einer Korngröße von 160 bis 540 Mikron, vorzugsweise 200 bis 400 Mikron enthalten.

3. Zusammensetzungen nach Anspruch 1 oder 2, **dadurch gekennzeichnet, dass** der Prozentanteil von Puzzolanerde zwischen 0,1 Gew.-% und 70 Gew.-% beträgt.

4. Zusammensetzungen nach Anspruch 3, **dadurch gekennzeichnet, dass** Puzzolanerde in einem Anteil von 0,5 bis 35 Gew.-%, vorzugsweise von 0,5 bis 30 %, vorzugsweise von D,5 bis 10 %, vorzugsweise von 0,5 bis 5 % und insbesondere von 0,5 bis 1 % enthalten ist.

5. Zusammensetzungen nach einem der Ansprüche 1 bis 4, **dadurch gekennzeichnet, dass** sie auch Bestandteile enthalten, die mit den kosmetischen, parapharmazeutischen oder pharmazeutischen Anwendungen kompatibel sind und Mineralstoffe, Wirkstoffe oder nützliche Stoffe enthalten, die von Puzzolanerde absorbiert werden können.

6. Zusammensetzungen nach Anspruch 5, **dadurch gekennzeichnet, dass** sie allein oder in Kombination Bestandteile enthalten, die gewählt werden unter:
- Extrakten von Heilpflanzen,
- Extrakten von aromatischen Pflanzen,
- essenziellen Ölen solcher Pflanzen,
- Thermalwasser und/oder Wasser aus (einer) Mineralquelle(n),
- Wässern oder Salzlösungen, denen speziell bestimmte Mineralstoffe zugesetzt wurden, zum Beispiel mit Magnesium und/oder Schwefel oder anderen Stoffen versetztes Thermalwasser.

7. Zusammensetzung nach Anspruch 5 oder 6, **dadurch gekennzeichnet, dass** die Pflanzenextrakte und essenziellen Öle gewählt werden unter:
dem essenziellen Ö1 von Kamille, Lavendel, Lavandinpflanze, Zypresse, Pinie, Tanne, Zitrone, Algen, Extrakten von Heilpflanzen, Extrakten von aromatischen Pflanzen, Extrakten aus Früchten und Gemüsen, Algenextrakten.

8. Zusammensetzungen nach Anspruch 5 oder 6, **dadurch gekennzeichnet, dass** sie allein oder in Kombination Meersalz, Ton, Mineralstoffe und Vitamine enthalten.

9. Zusammensetzungen nach einem der Ansprüche 5 bis 8, **dadurch gekennzeichnet, dass** das oder die verwendeten Mineralwässer und/ Quellwässer und/oder Salzlösungen in einem Anteil von 0 bis zur Menge QSP verwendet werden, d.h. bis zur Menge, die es ermöglicht, die kosmetische Zusammensetzung auf 100 Gew.-% aufzufüllen.

10. Zusammensetzungen nach einem der Ansprüche 5 bis 9, **dadurch gekennzeichnet, dass** sie mindestens 5 Ges.-% oder vorzugsweise mindestens 10 - 20 Gew.-% oder vorzugsweise 50 bis 70 - 100 Ges.-% Thermalwasser (Thermalwässer) oder Quellwasser (Quellwässer) im wässrigen Teil der Zusammensetzung enthalten.

11. Zusammensetzungen nach einem der Ansprüche 5 bis 10, **dadurch gekennzeichnet, dass** die Extrakte von aromatischen Pflanzen und Heilpflanzen und die essenziellen Öle in einem Anteil von (jeweils) 0 bis 1 oder 2 Gew.-%, vorzugsweise von 0 bis 3 oder 4 Gew.-% der kosmetischen Zusammensetzung enthalten sind.

12. Zusammensetzungen nach einem der Ansprüche 1 bis 11, **dadurch gekennzeichnet, dass** die Menge an Wasser (entweder entmineralisiertes Wasser oder entmineralisiertes und/oder Thermalwasser (Thermalwässer) und/oder Quellwasser (Quellwässer) und/oder Salzlösung) auf die Menge QSP 100 % eingestellt wird.

13. Zusammensetzungen nach einem der Ansprüche 1 bis 12, **dadurch gekennzeichnet, dass** sie in pharmazeutisch akzeptablen und kosmetologisch akzeptablen Formen mit exfolierender oder abrasiver Wirkung gegenüber der Haut vorliegen, die zur Anwendung sowohl durch Privatpersonen als auch durch kosmetologisch tätige Ärzte geeignet sind, wobei sie unter folgenden Formen gewählt werden:
- exfolierendes Duschgel, exfolierendes Gel für das Gesicht oder den Körper, exfolierendes Öl, exfolierendes Milch-Fluid, exfolierende schäumende Creme,
- exfolierende Seifen, exfolierende Cremeseifen,
- wobei diese Anwendungsformen Medikamente enthalten, die mit dem Rest der Rezeptur kompatibel sind.

14. Medikamente, **dadurch gekennzeichnet, dass** sie in der Form einer Zusammensetzung nach einem der Ansprüche 5 bis 13 vorliegen, darüber hinaus umfassend mindestens eine wirksame Dosis eines pharmazeutisch aktiven Stoffes, der kompatibel mit der Zusammensetzung und ihrer Art der Anwendung ist.

15. Verfahren zur Herstellung der Zusammensetzungen nach einem der Ansprüche 1 bis 14, **dadurch gekennzeichnet, dass** eine Mischung der Inhaltsstoffe vorgenommen wird, und zwar mit zwei Optionen:
- entweder die Puzzolanerde und die verschiedenen eventuellen Wirkstoffe, Thermalwässer, Quellwässer, essenziellen Öle, Pflanzenextrakte, Salzlösungen usw. werden gemeinsam gemischt,
- oder die Puzzolanerde wird vorab mit der Gesamtheit oder einem Teil dieser eventuellen Wirkstoffe durchtränkt, um die Absorption zu maximieren.

16. Zusammensetzung nach einem der Ansprüche 1 bis 13, **dadurch gekennzeichnet, dass** sie aus Folgendem besteht:
| EXFOLIERENDES GEL | | % |
|---|---|---|
| Entmineralisiertes Wasser oder Quellwasser (Quellwässer) und/oder Thermalwasser (Thermalwässer) | | |
| | QSP | 100 |
| Pflanzliche Wirkstoffe | | 20 |
| Carbomer | | 0,4 |
| Xanthangummi | | 0,8 |
| Puzzolanerde (200-400 µm) | | 2 |
| Essenzielles Ö1 | | 0,45 |
| Konservierungsstoffe | | 0,5 |
| Natriumhydroxid | | 0,2 |

17. Zusammensetzung nach einem der Ansprüche 1 bis 13, **dadurch gekennzeichnet, dass** sie aus Folgendem besteht:
| EXFOLIERENDES DUSCHGEL | | % |
|---|---|---|
| Entmineralisiertes Wasser oder Quellwasser (Quellwässer) und/oder Thermalwasser (Thermalwässer) | | |
| | QSP | 100 |
| Waschbasis | | 39 |
| Pflanzliche Wirkstoffe | | 16 |
| Puzzolanerde (260-400 µm) | | 5 |
| Konservierungsstoffe | | 0,5 |
| Essenzielles Ö1 | | 0,5 |
| Natriumchlorid | | 2,2 |
| Zitronensäure 10 % | | 0,25 |

18. Zusammensetzung nach einem der Ansprüche 1 bis 13, **dadurch gekennzeichnet, dass** sie aus Folgendem besteht:
| EXFOLIERENDE SCHÄUMENDE CREME | % | |
|---|---|---|
| Thermalwasser aus La Bourboule | QSP | 100 |
| Selbstemulgierende Basis | 4 | |
| Cetearylalkohol | 4 | |
| Pflanzliche Wirkstoffe | 5 | |
| Puzzolanerde (220-400 µm) | 1 | |
| Konservierungsstoffe | 0,5 | |
| Essenzielles Ö1 | 0,4 | |
| PEG-7 Glycerylcocoat | 1 | |

19. Zusammensetzung nach einem der Ansprüche 1 bis 13, **dadurch gekennzeichnet, dass** sie aus Folgendem besteht:
| EXFOLIERENDES SEIFENSTÜCK | % | |
|---|---|---|
| Pflanzliche Basis | QSP | 100 |
| Puzzolanerde (200-400 µm) | | 15 |
| Essenzielles Öl | | 0,5 |
Mit Vitaminen und Magnesium angereichertes EXFOLIERENDES SEIFENSTÜCK
| Mit 0,1% Vitamin B5, 0,1% Vitamin B1 und 0,1% | | |
|---|---|---|
| Magnesiumsulfat | | |
| Pflanzliche Basis | QSP | 100 |
| Magnesiumsulfat | | 2 |
| Vitamin B5 | | 0,1 |
| Vitamin B1 | | 0,1 |
| Puzzolanerde (200-400 µm) | | 15 |
| Essenzielles Ö1 | | 0,5 |

20. Zusammensetzung nach einem der Ansprüche 1 bis 13, **dadurch gekennzeichnet, dass** sie aus Folgendem besteht:
| Mit Algenextrakten und 1 % Meersalz angereichertes EXFOLIERENDES SEIFENSTÜCK | | |
|---|---|---|
| Pflanzliche Basis | QSP | 100 |
| Puzzolanerde (200-400 µm) | | 15 |
| Essenzielles Öl | | 0,5 |
| Meeresalgenextrakt | | 0,4 |
| Meersalz (Ile de Noirmoutier) | | 1 |
| Puzzolanerde (200-400 µm) | | 15 |
| Essenzielles Öl | | 0,5 |

21. Zusammensetzung nach einem der Ansprüche 1 bis 13, **dadurch gekennzeichnet, dass** sie aus Folgendem besteht:
| EXFOLIERENDES ÖL | % | |
|---|---|---|
| Ö1 | QSP | 100 |
| Öliges Geliermittel | 20 | |
| Flüchtiges Silikon | 10 | |
| Pflanzliche Wirkstoffe | 10 | |
| Puzzolanerde (200-400 µm) | 1,5 | |
| Konservierungsstoffe | 0,5 | |
| Essenzielles Ö1 | 0,45 | |

22. Zusammensetzung nach einem der Ansprüche 1 bis 13, **dadurch gekennzeichnet, dass** sie aus Folgendem besteht:
| EXFOLIERENDES DUSCH-CREMEGEL | % | |
|---|---|---|
| Entmineralisiertes Wasser | QSP | 100 |
| Waschbasis | 39 | |
| Selbstemulgierende Basis | 32 | |
| Puzzolanerde (200-400 µm) | 1,5 | |
| Konservierungsstoffe | 0,5 | |
| Essenzielles Ö1 | 0,3 | |
| Natriumchlorid | 2,2 | |
| Zitronensäure 10% | 0,25 | |

23. Zusammensetzung nach einem der Ansprüche 1 bis 13, **dadurch gekennzeichnet, dass** sie aus Folgendem besteht:
Mit Mineralsalzen und Magnesium im Ausmaß von 0,1 % angereichertes EXFOLIERENDES DUSCH-CREMEGEL
Lösung aus Magnesiumcarbonat, zinksulfat, Natriumsaccharinat und Kaliumcitrat mit Anteilen jedes Spurenelements von 0,1 % bezogen auf das Gewicht der gesamten Zusammensetzung
| | QSP | 100 |
|---|---|---|
| waschbasis | | 39 |
| Selbstemulgierende Basis | | 32 |
| Puzzolanerde (200-400 µm) | | 1,5 |
| Magnesiumcitrat | | 0,1 |
| Zinkcitrat | | 0,1 |
| Kaliumcitrat | | 0,1 |
| Konservierungsstoffe | | 0,5 |
| Essenzielles Ö1 | | 0,3 |
| Natriumchlorid | | 2,2 |
| Zitronensäure 10 % | | 0,25 |

24. Zusammensetzung nach einem der Ansprüche 1 bis 13, **dadurch gekennzeichnet, dass** sie aus Folgendem besteht:
| MILCH | |
|---|---|
| QSP 100 | |
| Selbstemulgierende Basis | 32 |
| Verdickungsmittel | 2,5 |
| Weichmacher | 10 |
| Vaselineöl laut Codex | 15 |
| Hydrophiles Geliermittel | 0,3 |
| Soda | 0,2 |
| Puzzolanerde (200-400 µm) | 2 |
| Konservierungsstoffe | 0,5 |
| Essenzielles Öl | 0,3 |
